(19) Europäisches Patentamt
European Patent Office
Office européen des brevets

(11) Veröffentlichungsnummer: **0 264 926 B1**

(12) # EUROPÄISCHE PATENTSCHRIFT

(45) Veröffentlichungstag der Patentschrift: **08.04.92**

(51) Int. Cl.$^5$: **C07D 319/12**

(21) Anmeldenummer: **87115384.7**

(22) Anmeldetag: **21.10.87**

(54) **Verfahren zur Herstellung und Reinigung thermolabiler Verbindungen.**

(30) Priorität: **24.10.86 DE 3636188**
**24.10.86 DE 3636187**
**28.07.87 DE 3724933**
**08.08.87 DE 3726499**

(43) Veröffentlichungstag der Anmeldung:
**27.04.88 Patentblatt 88/17**

(45) Bekanntmachung des Hinweises auf die Patenterteilung:
**08.04.92 Patentblatt 92/15**

(84) Benannte Vertragsstaaten:
**AT BE CH DE ES FR GB IT LI LU NL SE**

(56) Entgegenhaltungen:
**US-A- 3 442 871**

**CHEMICAL ABSTRACTS, Band 67, 1967, Seite 5031, Zusammenfassung Nr. 53702v, Columbus, Ohio, US; & DD-A-53 074 (P. ANDRATSCHKE et al.) 05-01-1967**

**CHEMICAL ABSTRACTS, Band 87, 1977, Seite 3, Zusammenfassung Nr. 39878p, Columbus, Ohio, US; A.P. BATALOV et al.: "Synthesis and polymerization of glycolide", & FIZ.-KHIM. OSN. SINT. PERERAB. POLIM. 1976, 1,35-6**

(73) Patentinhaber: **BOEHRINGER INGELHEIM KG**
**Postfach 200**
**W-6507 Ingelheim am Rhein(DE)**
(84) Benannte Vertragsstaaten:
**AT BE CH DE ES FR GB IT LI LU NL SE**

(73) Patentinhaber: **BOEHRINGER INGELHEIM INTERNATIONAL GmbH**
**Postfach 20**
**W-6507 Ingelheim am Rhein(DE)**
(84) Benannte Vertragsstaaten:
**GB**

(72) Erfinder: **Aigner, Michael, Dr. Dipl.-Ing.**
**Kapellenstrasse 7**
**W-6507 Ingelheim/Rhein(DE)**
Erfinder: **Dersch, Wolfgang, Dr.**
**Veit-Stoss-Strasse 17**
**W-6507 Ingelheim/Rhein(DE)**
Erfinder: **Reichert, Dieter, Dr.**
**Albrecht-Dürer-Strasse 17**
**W-6507 Ingelheim(DE)**
Erfinder: **Schwall, Horst, Dr.**
**Im Herzenacker 34**
**W-6535 Gau-Algesheim(DE)**
Erfinder: **Warth, Werner, Dr.**
**Matthias-Grünewald-Strasse 1**
**W-6507 Ingelheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die Erfindung betrifft ein Verfahren zur Herstellung und zur Reinigung thermolabiler viskoser Verbindungen, insbesondere von Glycoliden.

Im Sinne der Erfindung werden als Glycolide die cyclischen sechsgliedrigen Diester von α-Hydroxycarbonsäuren der allgemeinen Formel

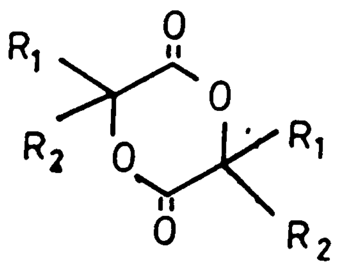

worin $R_1$ und $R_2$, die gleich oder verschieden sein können, Wasserstoff oder einen verzweigten oder unverzweigten Alkylrest mit bis zu 16 Kohlenstoffatomen bedeuten können, wie z.B. Methyl, Ethyl, n-Propyl, iso-Propyl, n-Butyl, iso-Butyl, tert.-Butyl u.a, angessehen.

Glyolide, insbesondere Glycolid (1,4-Dioxan-2,5-dion) selbst und Lactide, dienen u.a. als Ausgangsprodukte für die Herstellung biologisch abbaubarer medizinischer Hilfsmaterialien auf Polyesterbasis. Verwendet werden sowohl Polyglycolid (1,4-Dioxan-2,5-dion) und Polylactid selbst, als auch die Copolymeren von Lactiden mit anderen Glycoliden, insbesondere 1,4-Dioxan-2,5-dion und weiteren polymerisationsfähigen Reaktionspartnern.

Die Herstellung von Glycoliden ist in der Literatur beschrieben.

Bekannt sind z.B. Herstellungsverfahren für Glycolid (1,4-Dioxan-2,5-dion) auf der Basis von Glykolsäure (DE-OS'en 1668 993 und 1668 994), Halogenessigsäuren (F. Andreas et al., J. pr. chem. 18, 141 (1962) und deren Salzen, Polyglykolsäure, Glykolsäureestern (FR 14 85 302), Chloracetyl-glykolsäuresalzen (US 37 63 190) und Chloracetylpolyglykolsäure (J. Am. Chem. Soc. 76, 754 (1954), oder allgemein Herstellverfahren auf der Basis von α-Halogencarbonsäuren, α-Hydroxycarbonsäuren und deren Derivate, wie z.B. α-Hydroxycarbonsäureester und Poly-α-hydroxycarbonsäuren. Im Falle der monomeren Edukte wird zunächst ein Polyester gebildet, der anschließend in situ oder nach Isolierung der weiteren Umsetzung zu dem Dimeren unterzogen wird. Diese Umsetzung besteht in einer Thermolyse bei 200 - 300° C unter Zusatz von Metallen, Metalloxiden oder Salzen. Die hierbei gebildeten Dimere werden - vorzugsweise im Vakuum - abdestilliert. Das Destillat wird in mehrfach durchzuführenden Reinigungsschritten, wie z.B. Kristallisation, aus geeigneten Lösungsmitteln in eine stabile Form überführt. Eine derartige Verfahrensweise ist beispielsweise für das Lactid in dem britischen Patent 1 007 347 beschrieben.

Bei der dort offenbarten Thermolyse handelt es sich, bedingt durch die Eigenschaften von Ausgangsmaterial und Reaktionsprodukt, um eine gefährliche Reaktion, die aufgrund einer möglichen Explosionsgefahr als eine Reaktion mit hohem Sicherheitsrisiko einzuordnen ist. Die thermodynamische Untersuchung der Reaktion ergibt, daß die zur Bildung von Glycolid aus Polyglycolsäure oder von Lactiden aus Polymilchsäuren erforderliche Reaktionstemperatur im thermischen Zersetzungsbereich der Verbindungen zu gasförmigen Folgeprodukten liegt.

Die Exothermie der Zersetzungsreaktion wird durch die Verdampfungsenthalpie der gebildeten Glycolide kompensiert. Hieraus folgt, daß die Reaktion dann zur Thermoexplosion des Reaktionsgemisches führen kann, wenn die Verdampfung der Glycolide - aus welchem Grunde auch immer - nicht mehr gewährleistet ist, wie z.B. bei Ausfall des Vakuums, Verstopfen der Leitungen etc.. Nachstehend sei der Ablauf des konventionellen Thermolyseprozesses unter Satzbedingungen am Beispiel von Glycolid bzw. Lactid skizziert.

Die als Ausgangsprodukt dienende niedermolekulare Polymilchsäure (Polyglycolsäure) wird mit dem Katalysator gemischt, in die Reaktionsapparatur eingebracht und auf die erforderliche Reaktionstemperatur von 240-260° C aufgeheizt. Während des Aufheizvorganges schmilzt das Ausgangsprodukt und bei 240° C beginnt das Lactid (Glycolid) abzudestillieren. Das Reaktionsgemisch färbt sich nach kurzer Zeit dunkel und geht in einen viskosen Zustand über. Ab diesem Zeitpunkt ist das Reaktionsgemisch nicht mehr einwandfrei homogenisiert. Das inhomogene Reaktionsgemisch neigt zu unkontrollierter Zersetzung, da sich lokale Überhitzungsherde ausbilden können. Der nach beendeter Reaktion im Reaktionsgemisch verbleibende

Reaktionsrückstand muß durch längeres Erhitzen in konzentrierter Natronlauge aus dem Reaktionsgefäß entfernt werden. Infolge der inhomogenen Reaktionsbedingungen werden bei der satzweisen Thermolyse schwankende Qualitäten und Ausbeuten erzielt. Die Ausbeute an Destillat, d.h. an Rohlactid, beträgt 70 - 90 % d. Th. t.q. Nach der Aufarbeitung des Destillats durch Fällung in einem geeigneten organischen Lösungsmittel, wie z.B. Alkoholen ($C_1$ bis $C_4$) oder halogenierten Kohlenwasserstoffen (z.B. Tetrachlorkohlenstoff) wird eine Ausbeute von 40 - 60 % d. Th. erhalten. Die DE-OS 15 43 958 gibt beispielsweise bei der Thermolyse eine Ausbeute von 56 % an, wobei nachfolgende Reinigungsschritte noch nicht berücksichtigt sind.

Überraschenderweise können die vorstehend beschriebenen Nachteile des literaturbekannten Herstellprozesses mit dem erfindungsgemäßen Verfahren vermieden werden.

Somit betrifft die vorliegende Erfindung ein Verfahren zur Destillation thermolabiler Verbindungen, wobei man (a) die zu destillierende Verbindung aus einem Vorratsbehälter in das Strömungsrohr eines evakuierbaren selbstreinigenden Doppelschneckenextruders versehen mit getrennt regelbaren Heizsegmenten, mindestens einer Brüdenableitung und einem davon getrennten Austrag, einspeist und mittels Zwangsförderung durch Heizsegmente mit ansteigender Temperatur auf Siedetemperatur erhitzt, (b) im Bereich der Siedetemperatur das Destillat über die Brüdenableitung(en) abzieht, anschließend kondensiert und (c) den höhersiedenden Rückstand durch Zwangsförderung aus dem Destillationsbereich in einen Rückstandsbehälter austrägt. - In einer besonderen Ausführungsform des oben beschriebenen Verfahrens werden die Heizsegmente zwischen 15 und 400 ° C temperiert.

Eine weitere Ausführungsform des oben beschriebenen Verfahrens besteht darin, daß die Temperatur der Heizsegmente nach den Brüdenableitungen niedriger ist als unterhalb der Brüdenableitungen.

Die vorliegende Erfindung betrifft ferner die Verwendung eines evakuierbaren Doppelschneckenextruders, versehen mit getrennt regelbaren Heizsegmenten, mindestens einer Brüdenableitung für das Destillat und davon getrenntem Austrag für den Destillationsrückstand zur Destillation bei der Herstellung oder Reinigung thermolabiler Verbindungen.

Die vorliegende Erfindung betrifft weiterhin ein Verfahren zur Herstellung von einem Glycolid der allgemeinen Formel

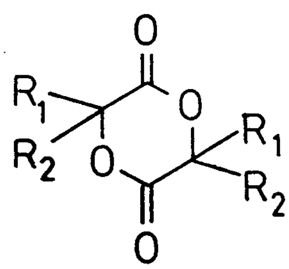

worin $R_1$ und $R_2$ unabhängig voneinander Wasserstoff oder einen verzweigten oder unverzweigten Alkylrest mit bis zu 16 Kohlenstoffatomen bedeuten können, durch Thermolyse der entsprechenden niedermolekularen Poly-$\alpha$-hydroxycarbonsäure oder des aus einem $\alpha$-Halogencarbonsäuresalz in situ gebildeten Polyesters in Gegenwart eines Metall, Metalloxids oder Salzes als Katalysator wobei man

(a) die zu destillierende Verbindung aus einem Vorratsbehälter in das Strömungsrohr eines evakuierbaren, selbstreinigenden Doppelschneckenextruders, versehen mit getrennt regelbaren Heizsegmenten, mindestens einer Brüdenableitung und einem davon getrennten Austrag, einspeist und mittels Zwangsförderung durch Heizsegmente mit ansteigender Temperatur auf Siedetemperatur, gegebenenfalls unter Schutzgas und/oder vermindertem Druck erhitzt,

(b) im Bereich der Siedetemperatur das Destillat über die Brüdenableitung(en) abzieht, anschließend kondensiert und

(c) den höhersiedenden Rückstand durch Zwangsförderung aus dem Destillationsbereich in einen Rückstandsbehälter austrägt.

Eine besondere Ausführungsform des oben beschriebenen Verfahrens besteht darin, daß der Temperaturgradient von 15 bis 280 ° C ansteigt und der Druck 0.1 bis 5 mbar beträgt.

Insbesondere betrifft die vorliegende Erfindung ein Verfahren zur Herstellung von 1,4-Dioxan-2,5-dion wobei als Ausgangsverbindung niedermolekulare Polyglycolide oder Halogenessigsäuresalze in Gegenwart eines Katalysators eingesetzt werden.

Daneben betrifft die vorliegende Erfindung ein Poly-(1,4-dioxan-2,5-dion), das eine inhärente Viskosität von > 1,4 [dl/g], gemessen in Hexafluorisopropanol bei 30 ° C, aufweist.

Selbstreinigende Doppelschneckenextruder mit aufgesetzen Brüdenableitungen sind bekannt und finden in der Kunststoffherstellung vielfältige Anwendung. Die Geräte sind in der Regel nach dem Baukastenprinzip konstruiert und werden von einer Reihe von Maschinenbaufirmen in vergleichbarer Ausstattung auf dem Markt angeboten. Während in der kunststoffverarbeitenden Industrie Doppelschneckenextruder zum Homogenisieren von Kunststoffgranulaten, evtl. unter Zumischung von Hilfsstoffen wie Füllmaterialien oder Farbstoffen sowie zur Erzeugung des notwendigen Vordruckes am formgebenden Werkzeug dienen, wird im Falle der Herstellung von Glycoliden der Extruder als kontinuierlich arbeitender Chemiereaktor eingesetzt.

Erfindungsgemäß ergeben sich gegenüber bekannten Herstellungsverfahren die folgenden Vorteile:
Die Reaktionsmasse wird infolge des kleinen Thermolysevolumens gering gehalten, Störungen oder Gefahren durch Druckaufbau während der Reaktion sind deshalb unbedeutend und bedürfen keiner besonderen Maßnahmen. Die Doppelschnecke des Extruders sorgt zum einen für eine perfekte Homogenisierung des Reaktionsgemisches und somit für eine über den Querschnitt des Strömungsrohres einheitliche Reaktionstemperatur und zum anderen für den Zwangsaustrag des durch Nebenreaktion stets entstehenden Polymermaterials, weiterhin erfolgt durch eine geeignete Anordnung der Doppelschnecke eine ständige Selbstreinigung des Reaktors, wodurch ein Verkleben des Reaktionsgemisches mit Teilen des Reaktors vermieden wird, da der Rückstand durch die Doppelschnecke zwangsweise in den Rückstandsbehälter transportiert wird. Dadurch wird eine kontinuierliche Fahrweise der Thermolyse ermöglicht.
Das nachfolgende Schema veranschaulicht das Prinzip der Reaktionsführung im zwangsfördernden Strömungsrohr eines Doppelschneckenextruders.

Der Aufbau eines solchen Extruders ist schematisch in Abb. 1 dargestellt.

Der Extruder besitzt Vorrichtungen, die eine getrennte Temperierung der einzelnen Segmente erlauben, so daß ein Temperaturgradient aufgebaut werden kann, wobei die Temperierung beispielsweise gemäß folgendem Schema vorgenommen wird:

| Gehäuse-Nr. | Temp ° C |
|---|---|
| 0 | 15 |
| 1 | 130 |
| 2 | 150 |
| 3 | 240 |
| 4 | 280 |
| 5 | 280 |
| 6 | 280 |
| 7 | 280 |
| 8 | 280 |
| 9 | 250 |

Die Anzahl der Heizsegmente kann selbstverständlich variiert werden, wenn eine differenziertere Temperaturführung erwünscht ist.

Über den Gehäusen Nr. 7 und 8 sind die Brüdenableitungen angeordnet, mit denen das Produkt als Destillat aufgefangen wird. Weiterhin besteht die Apparatur aus einem Vorratsbehälter und einem Rückstandsbehälter sowie aus Vorrichtungen zur Aufrechterhaltung eines Vakuums.
Über eine Dosierschnecke wird das Ausgangsmaterial in den eigentlichen Reaktor gefördert, in dem eine Doppelschnecke einerseits die Zwangsförderung durch den Reaktor bewirkt, andereseits eine homogene Vermischung des plastischen Reaktionsgemisches wie auch die Selbstreinigung während des gesamten Reaktionsverlaufs gewährleistet.

Verfahrensbescheibung:

Die in die Depolymerisationsreaktion eingesetzten Poly-hydroxycarbonsäuren werden wie in der Literatur beschrieben, vergl. zB. Chem. Ber. 58, 1307 (1925), hergestellt:
Beispielsweise werden handelsübliche optisch aktive Milchsäuren [R], [S] oder racemische Milchsäure im Vakuum bis zu einer Sumpttemperatur von 200 ° C durch Destillation entwässert. Dabei entstehen oligomere Milchsäuren. Diese Milchsäuren sind bei erhöhter Temperatur flüssig und erstarren beim Abkühlen auf Raumtemperatur zu glasartigen Massen, die mit den üblichen mechanischen Methoden zerkleiniert werden können. Die auf diesem Wege gewonnenen Pulver oder Granulate werden mit einem geeigneten Katalysator gemischt und mittels einer Feststoffdosiereinrichtung in den Produkteintrag des Extruders eingebracht.

Die Doppelschnecke fördert die Reaktionsmasse durch das Strömungsrohr. Im Rückstau der Schnecke wird durch geeignete Wahl von Drehzahl, Feed und Temperatur eine derartige Konsistenz der Reaktionsmasse eingestellt, daß das auf der Destillationsseite anliegende Vakuum von 2-5 mbar aufrechterhalten wird. Der Rückstau im Strömungsrohr kann durch eine geeignete Wahl der Schneckenkonfiguration bewirkt werden, z B. durch Änderung der Schneckensteigung, bzw. durch Umkehr des Fördersinns. (In Abb. 1 ist die Schneckenkonfiguration einer Schnecke schematisch dargestellt; dem Fachmann ist die technische Realisation einer entsprechenden selbstreinigenden Doppelschnecke hinreichend bekannt). Die Reaktionsmasse durchläuft dann einen Temperaturgradienten von 130-280 ° C. Hierbei findet die Thermolyse von niedermolekularem Polyglykolid, zu Glykolid statt. Unter den Brüdenöffnungen erfolgt die Verdampfung, das erhaltene Destillat wird in einen temperierten Vorratsbehälter abgeführt, der verbleibende Rückstand liegt bei ca. 5 Gew.-% der eingetragenen Menge und wird zwangsweise durch die Schnecken in den Rückstandsaustragsbehälter gefördert, so daß ein kontinuierlicher Betrieb ermöglicht wird. Bei der Durchführung des Verfahrens erweist es sich als vorteilhaft, wenn die Heizsegmente nach den Brüdenableitungen (in Abb. 1, Heizsegment 9) eine niedrigere Temperatur als die an den Brüdenableitungen aufweisen; hierdurch wird gewährleistet, daß das durch Nebenreaktion entstehende Polymermaterial verfestigt und ausgetragen wird. Wenn es gewünscht wird, können weitere Brüdenableitungen in Bereichen niederer Temperaturen aufgesetzt werden, um niedrigsiedende Verunreinigungen zu entfernen.

Das vorstehend geschilderte Verfahren ist im technischen Maßstab durchführbar. Die Ausbeute an Destillat (Rohglycolid) beträgt ca. 95,0 % d.Th. t. q..

Beschreibung der Apparatur

Der Extruder wird mit einer Dosierschnecke zum Feststoffeintrag an der Eintragseite, des weiteren mit einem beheizten Brüdenrohr, einem Kondensator, zwei wechselseitig schaltbaren Vakuumvorlagen zur Vakuumdestillation und einem vakuumdichten Austragsbehälter, der den Reaktionsrückstand aufnimmt, ausgestattet. Einzelheiten der Apparatur können dem beigefügten Verfahrensfließbild (Abb. 1) entnommen werden. Zusätzlich erhält die Appartur Vorrichtungen zur Herstellung und Aufrechterhaltung des notwendigen Vakuums von 2 bis 5 mbar. Die Segmente 0 bis 9 bedeuten Teile des Reaktors, die unabhängig voneinander beheizt werden können, womit der notwendige Temperaturgradient hergestellt werden kann.

Die Schnecke ist so konzipiert, daß infolge des plastischen Materials ein Vakuum in einem bestimmten Abschnitt des Reaktors problemlos aufrechterhalten werden kann. Abb. 1 verdeutlicht den Druckverlauf innerhalb des Reaktors.

Nach dem erfindungsgemäßen Verfahren werden bevorzugt Glycolide der allgemeinen Formel I worin $R_1$ und $R_2$ die nachfolgend aufgeführte Bedeutung aufweisen, hergestellt.

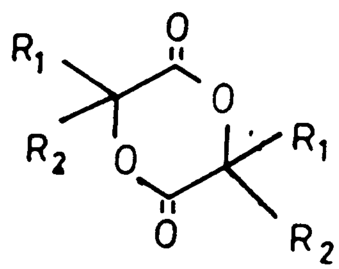

| | | | | | |
|---|---|---|---|---|---|
| $R_1$ | H | H | $CH_3$ | $C_2H_5$ | $CH(CH_3)_2$ |
| $R_2$ | H | $CH_3$ | $CH_3$ | H | H |

| | | | | | |
|---|---|---|---|---|---|
| $R_1$ | $C_2H_5$ | $n\text{-}C_4H_9$ | $C(CH_3)_3$ | $nC_5H_{11}$ | $nC_7H_{15}$ |
| $R_2$ | $CH_3$ | H | H | H | H |

| | | | | |
|---|---|---|---|---|
| $R_1$ | $n\text{-}C_{12}H_{25}$ | $nC_{13}H_{27}$ | $nC_{14}H_{29}$ | $nC_{15}H_{31}$ |
| $R_2$ | H | H | H | H |

| | |
|---|---|
| $R_1$ | $nC_{16}H_{33}$ |
| $R_2$ | H |

Zur Herstellung von Glycolid (1,4-Dioxan-2,5-dion) wird bei dem erfindungsgemäßen Verfahren beispielsweise als Ausgangsmaterial ein niedermolekulares (oligomeres) Polyglycolid bzw. ein Halogenessigsäuresalz eingesetzt und zusammen mit einem geeigneten Katalysator in dem Vorratsbehälter (s. Abb. 1) vorgelegt. Die Herstellung von niedermolekularem Polyglykolid ist allgemein bekannt, so z.B. durch Erhitzen von Halogenessigsäuren und/oder Natriumchloracetat in Xylol. Geeignete Katalysatoren zur Depolymerisation sind ebenfalls Stand der Technik, beispielsweise Zinn und Zink, bzw. ihre Verbindungen. Ein bevorzugter Katalysator ist Zinkoxid. Im allgemeinen werden zwischen 0.01 und 4 Gew. % Katalysator eingesetzt. Die Edukte werden dann mittels einer Dosierschnecke in den Extruder eingebracht und wie zuvor beschrieben umgesetzt.

Nach dem vorstehend geschilderten Verfahren kann die Thermolyse in den technischen Maßstab übertragen werden. Die Ausbeute Destillat (Rohglykolid) beträgt 95 % d.Th. t.q.. Nach Aufarbeitung durch Fällung in Isopropanol ergibt sich eine Ausbeute von 81 % d.Th. Die Ausbeutesteigerung gegenüber dem Batch-Verfahren beträgt im Mittel ca. 60 %. Das Produkt wird einem weiteren Reinigungsprozeß zugeführt, der zu einer Glykolidqualität führt, die zur Herstellung hochmolekularer Polyester, wie sie z.B. zur Herstellung von chirurgischem Nahtmaterial Verwendung finden, geeignet ist.

Das neue, in einem selbstreinigenden, zwangsfördernden Strömungsrohr (Doppelschneckenextruder) durchgeführte Verfahren zeichnet sich gegenüber den in der Literatur beschriebenen Verfahren insbesondere durch folgende Vorteile aus:

- problemlose Durchführbarkeit der Reaktion aufgrund des zwangsweisen Austrags von gebildetem Polymermaterial,
- höhere Ausbeute
- bessere Produktqualität
- hohe Sicherheit bei der Durchführung dieser Reaktion hoher Gefahrenklasse, aufgrund des sehr kleinen Thermolysevolumens im Strömungsrohr und der dadurch bedingten sehr geringen Reaktionsmasse,

- hohe Umweltfreundlichkeit. Es fallen -bedingt durch die hohe Ausbeute und die spezielle Verfahrenstechnik- nur minimale Mengen festen Abfalls an. Die großen Mengen stark alkalischer Abwässer, die beim Batch-Prozeß entstehen, werden vermieden.

Durch das neue, kontinuierlich arbeitende Verfahren wird nunmehr die Herstellung von Glycoliden in einem konventionell ausgestattetem Chemiebetrieb ohne besondere, aufwendige Sicherheitseinrichtungen möglich.

Das vorgenannte Herstellverfahren ist auch zur Destillation chemischer Verbindungen geeignet.

Die Destillation ist eine weithin angewandte Methode zur Reinigung bzw. Trennung organischer und anorganischer chemischer Substanzen (vergl. R. Billet, Industrielle Destillation, Verlag Chemie, Weinheim, 1973).

Destillationen werden häufig im Vakuum durchgeführt, um durch Verminderung des Siedepunktes die thermische Belastung der Substanzen zu verringern. Diverse technische Ausführungen sind Stand der Technik, u.a. Apparaturen, um die Vakuumdestillation im kontinuerlichen Verfahren durchzuführen. Apparaturen, die eine möglichst schonende Vakuumdestillation chemischer Substanzen ermöglichen, sind z.B. Dünnschichtverdampfer und Fallfilmverdampfer verschiedener Bauart und Ausführung. Diese Destillationsverfahren werden sowohl zur Substanzdestillation im eigentlichen Sinne benutzt, als auch, um leichter flüchtige Nebenbestandteile wie Lösungsmittel oder im Falle polymerer Substanzen Restmonomere abzutrennen. Das gewünschte Produkt befindet sich in diesen Fällen im Destillationsrückstand. In den Fällen, wo durch Destillation leichtflüchtige Anteile aus hochviskosen Gemischen entfernt werden sollen, bedient man sich in der Technik in Ausnahmefällen auch der Schraubenverdampfer (vergl. Ullmann, Enzyclopädie der technischen Chemie, Band 2, Seite 658 ff). Die Entgasung von Monomoren aus Polymermaterial mit Hilfe von Schneckenverdampfern wird beispielsweise von K.-M. Hess in Chem.-Ing.-Techn. 51 (3), 245 (1979) beschrieben.

Die Destillation von chemischen Substanzen findet im allgemeinen dort ihre Grenzen, wo die Substanzen nicht mehr unzersetzt verdampfbar sind und durch den Zersetzungsanteil hochsiedende und hochviskose oder feste, häufig polymere Nebenprodukte gebildet werden. Diese Nebenprodukte führen bei technischen Destillationsprozessen zu einer Reihe von Problemen, die ihre Ursache in der Belagbildung (Fouling) an den Oberflächen der Destillationsapparate und Wärmeaustauscher haben. Durch Randbelagbildung wird der Wärmeübergang verschlechtert und die gleichmäßige Verdampfung der Substanz findet nicht mehr statt. Dieser Prozeß des Foulings ist besonders bei allen Arten von kontinuierlichen Verdampfern, besonders aber bei kontinuierlichen Fall- oder Kletterfilmverdampfern der verschiedenen Bauarten von großem Nachteil.

Überraschenderweise konnten die vorstehend genannten bekannten Nachteile bei der Destillation von thermolabilen Verbindungen unter Anwendung herkömmlicher Verfahren dadurch beseitigt werden, daß die zu destillierende Substanz in einem Reaktor (Strömungsrohr) mit ansteigendem Temperaturgradienten unter Zwangsförderung destilliert wird.

Das erfindungsgemäße Verfahren zeichnet sich dadurch aus, daß chemische Verbindungen, die bei der Destillation zur Zersetzung neigen, auch bei höheren Temperaturen, gegebenenfalls im Vakuum, mit ausgezeichneten Ausbeuten und verrringertem Sicherheitsrisiko destillierbar sind.

So können eine Reihe von Substanzen, insbesondere organische Verbindungen, bei hoher Temperatur im Hochvakuum innerhalb des thermischen Zersetzungsbereiches der Substanzen destilliert werden, die in Verdampfern herkömmlicher Art nicht oder nur mit schlechter Ausbeute und/oder nur unter hohem Sicherheitsrisiko destillierbar sind.

Der Aufbau eines zur Destillation verwendbaren Extruders ist bereits im wesentlichen in Abb. 1 dargestellt, jedoch gibt es Unterschiede in der Temperierung der einzelnen Segmente.

Abbildung 2 zeigt schematisch den Aufbau eines Extruders der für Destillationen geeignet ist.

Der Extruder besitzt Vorrichtungen, die eine getrennte Temperierung einzelner Segmente erlauben, so daß ein Temperaturgradient aufgebaut werden kann, wobei die Temperierung beispielsweise gemäß folgendem Schema vorgenommen wird:

| Gehäuse-Nr. | Temp ° C |
|---|---|
| 0 | 15 |
| 1 | 50 |
| 2 | 70 |
| 3 | 140 |
| 4 | 190 |
| 5 | 200 |
| 6 | 200 |
| 7 | 200 |
| 8 | 190 |
| 9 | 190 |

Über den Gehäusen Nr. 7 und 8 sind die Brüdenableitungen angeordnet, mit denen das als Destillat aufgefangen wird. Weiterhin besteht die Apparatur aus einem Vorratsbehälter und einem Rückstandsbehälter sowie aus Vorrichtungen zur Aufrechterhaltung eines Vakuums. Aus dem Vorratsbehälter wird die zu destillierende Substanz in das Strömungsrohr eingespeist, wobei sie infolge der Zwangsförderung, z.B. durch eine gleichlaufende Doppelschnecke, durch Segmente mit ansteigender Temperatur auf Siedetemperatur erhitzt wird. Die Temperaturführung ist von verschiedenen Faktoren abhängig, wie z.B. Konstruktion des Reaktors (Extruders), Geschwindigkeit und Menge und Art des eingetragenen Materials, etc. Im Bereich der Siedetemperatur wird das Destillat abgezogen, während der höhersiedende Rückstand infolge der Zwangsförderung aus dem Destillationsbereich entfernt wird. Zur Duchführung des Verfahrens ist es vorteilhaft, wenn die Heizsegmente nach den Brüdenableitungen eine Temperatur unterhalb der Siedetemperatur des Destillats aufweisen, so daß der Destillationsrückstand fest wird, wenn er infolge der Zwangsförderung ausgetragen wird. Im Rückstau der Schnecke (vgl. Abb. 2) kann durch geeignete Wahl von Drehzahl, Einspeismenge und Temperatur eine derartige Konzistenz der Reaktionsmasse eingestellt werden, so daß auf der Destillationsseite ein Unterdruck aufrechterhalten werden kann. Der Rückstau im Strömungsrohr kann durch eine geeignete Wahl der Schneckenkonfiguration bewirkt werden, z.B. durch Änderung der Schneckensteigung, bzw. durch Umkehr des Fördersinns. (In Abb. 2 ist die Schneckenkonfiguration einer Schnecke schematisch dargestellt; dem Fachmann ist die technische Realisation einer entsprechenden Doppelschnecke hinreichend bekannt.) Das erhaltene Destillat wird kondensiert und gegebenenfalls weiterverarbeitet.

Die Anzahl der Heizsegmente, die Temperaturführung, Geschwindigkeit der Schnecke, Ort und Zahl der Brüdenableitungen können selbstverständlich auf das spezielle Destillationsproblem abgestimmt werden.

Das erfindungsgemäße Verfahren ist nicht ausschließlich auf die Destillation von flüssigen bzw. zähflüssigen Stoffen beschränkt. Aufgrund der Zwangsförderung der Substanz können auch plastische oder feste Verbindungen destilliert bzw. sublimiert werden. Es ist hier selbstverständlich, daß die Temperierung der Brüdenableitung wie auch ihre Konstruktion so ausgelegt ist, daß die Kondensation des Produktes nicht zu einer Verstopfung der Apparatur führt und beispielsweise erst in einer geeigneten Kühlfalle erfolgt.

Die Temperatur der Heizsegmente wird durch das verwendete Heizmedium bestimmt. Es folgt die Temperierung der Segmente durch Öl. Liegt die maximale Heizsegmenttemperatur bei ca. 300 ° C, bei der Verwendung einer elektrischen Heizung können ca. 400 ° C erreicht werden. Solche Heizsysteme sind im Handel bereits erhältlich. Es ist jedoch denkbar für spezielle Problemstellungen auch Sonderanfertigungen mit höher temperierbaren Segmenten zu verwenden. Zur Absenkung der Siedepunkte kann die Destillation bei vermindertem Druck ausgeführt werden. Konstruktionsbedingt können Drücke bis zu einer unteren Grenze von ca. 0.5 mbar eingestellt werden.

Das vorstehend beschriebene Destillationsverfahren eignet sich nicht nur für den Labormaßstab, sondern auch für Destillationen im technischen Maßstab.

Das neue Verfahren erlaubt die Hochtemperaturdestillation von schwerflüchtigen organischen Verbindungen nach einer kontinuierlichen Verfahrensweise. Das erfindungsgemäße Destillationsverfahren eignet sich besonders zur Reinigung von Verbindungen, bei denen bei herkömmlichen Destillationsprozessen sich eine höher siedende Komponente als unerwünschtes Nebenprodukt im Sumpf anreichert. Das Verfahren ist ebenfalls dazu geeignet Verbindungen von festen Rückständen abzudestillieren.

Damit ist die Möglichkeit gegeben, Substanzen die unter konventionellen Bedingungen wegen ihrer Temperaturlabilität nicht destilliert werden können gefahrlos und in hoher Ausbeute zu destilieren und damit zu reinigen.

In den Fällen, wo durch das neue Verfahren eine - zum Teil mehrfache - Umkristallisation des Produkts ersetzt werden kann, trifft als weiterer Vorteil die Umweltfreundlichkeit des Destillationsverfahrens hinzu:

Es fallen keine oder nur sehr geringe Rückstände an, es werden keinerlei Hilfschemikalien benötigt, Emissionen treten nicht auf,
das Abwasser wird nicht belastet.

Die nachfolgenden Beispiele sollen das erfindungsgemäße Verfahren näher erläutern.

1: Apparatur

Doppelschneckendosiergerät für Feststoffe Doppelschneckenextruder mit Brüdenabführung und Vakuumanschluß

Glaskondensator mit Wärmeträger beschickt Vakuumvorlagen mit Doppelmantel Rückstandsaustragsbehälter mit Stickstoffausgleich und Vakuumanschluß

2. Durchführung des Verfahrens

Der Extruder wird entsprechend den in den Beispielen angegebenen Temperaturgradienten temperiert.

Die Brüdenabtelung wird auf Solltemperatur temperiert, die Doppelmantelglasvorlagen werden mit Wärmeträgermedium auf eine Temperatur oberhalb der Schmelztemperatur der entsprechenden Verbindung beheizt.

Nach Erreichen der Solltemperatur wird der Extruder mit der Normeinspeisung, wie im Beispiel genannt, und der Normschneckendrehzahl beschrieben. Während der Destillation beträgt der Druck 0,5 bis 1025 mbar, vorzugsweise 2 - 10 mbar. Das Destillat wird nach Belüftung der Vakuumvorlagen mit Stickstoff in eine Thermogefäß abgelassen und einer geeigneten Aufarbeitungsmethode, wie z.B. einer Granulation in einem Nichtlöser oder einer Kühlerstarrung mittels Band oder Walze, zugeführt.

Das erfindungsgemäße Verfahren ist besonders zur Reinigung von Glycolid (1,4-Dioxan-2,5-dion) geeignet, wobei man ein Glycolid von sehr hoher Reinheit, das ohne weitere Reinigungsmaßnahmen zur Polymerisation eingesetzt werden kann, erhält.

Das nach dem erfindungsgemäßen Verfahren erhaltene kondensierte Destillat an reinem Glycolid wird anschließend in einem Nichtlösemittel, z.B. Petrolether, gefällt. Die so erhaltene Kristallsuspension wird abgetrennt und getrocknet. Es ist ebenfalls möglich und im technischen Maßstab erstrebenswert, das Destillat über eine Kühlvorrichtung, wie z.B. eine Kühlwalze oder ein Kühlband, direkt erstarren zu lassen. Der Umweg über die Granulierung in einem inerten Lösungsmittel und der anschließende Trenn- und Trocknungsschritt wird dadurch vermieden.

Wenn es gewünscht wird oder es erforderlich erscheint, können im Bereich niederer Temperaturen niedrigsiedende Verunreinigungen des rohen Glykolids durch zusätzliche Brüdenableitungen entfernt werden.

Das erfindungsgemäße Verfahren ermöglicht die kontinuierliche Destillation von rohem Glykolid in einem technischen Maßstab, wobei zwischen 95 und 98 Gew.-% (bezogen auf eingesetztes rohes Glykolid) hochreines Glykolid erhalten werden, wie es nach bisher bekannten Verfahren in dieser Reinheit nicht in einem technischen Maßstab hergestellt werden konnte.

Die aus dem Stand der Technik bekannten Reinigungsverfahren, sei es durch Umkristallisation oder durch Sublimation, ergeben ein Glykolid, das sich von dem, nach dem erfindungsgemäßen Verfahren hergestellten Glykolid in seinen Stoffeigenschaften unterscheidet. So bedingen beispielsweise satzweise Destillationsprozesse eine größere thermische Belastung des Glykolids und führen zu einem höheren Anteil an Zersetzungsprodukten, die auch bei einer Sublimation an der Kühlfläche abgeschieden werden und sich nicht mehr aus dem Glykolid entfernen lassen.

Während ein nach bekannten Reinigungsverfahren hergestelltes Glykolid (1,4-Dioxan-2,5-dion) sich zu einem Polyglykolid mit einer inhärenten Viskosität von ca. 1[dl/g]polymerisieren läßt, erhält man unter identischen Polymerisationsbedingungen (z.B. $SnCl_2 \times H_2O$, Laurylalkohol) aus einem nach dem erfindungsgemäßen Verfahren hergestellten Glykolid ein Polyglykolid mit einer inhärenten Viskosität von > 1.4 [dl/g]. (Die inhärenten Viskositäten werden in Hexafluorisopropanol bei 30 °C bestimmt).

Beispiel 1:

Kontinuierliche Herstellung von Glykolid aus niedermolekularem Polyglykolid mit katalytischen Mengen Zinkoxid

Niedermolekulares Polyglykolid wird mit 2 Gew.-% Zinkoxid versetzt und in einer geeigneten Mischapparatur wie z.B. einem Rhönradmischer homogenisiert.

| Gehäuse Nr. | 0 | 1 | 2 | 3 | 4 | 5 | 6 | 7 | 8 | 9 |
|---|---|---|---|---|---|---|---|---|---|---|
| Temperatur (°C) | 15 | 130 | 150 | 240 | 280 | 280 | 280 | 280 | 280 | 250 |

Der Extruder wird entsprechend obiger Tabelle (vergl. auch Abb. 1) temperiert, die Brüdenableitung wird auf 180°C, die Vakuumvorlagen zur Aufnahme des Destillats auf 80°C aufgeheizt.

Nach Erreichen der Solltemperatur wird der Extruder mit einer Drehzahl von 1,67/sec. und einem Feed von 3,0 kg/Stunde niedermolekularem Polyglykolid bei einem Druck von-2-5 mbar betrieben.

Die Vakuumvorlagen werden nach jeweils 60 Minuten entleert, das Destillat wird aufgearbeitet. Die Ausbeute an Destillat beträgt 95% d.Th. Die Aufarbeitung des Destillats erfolgt konventionell durch satzweises Umfällen aus Isopropanol in einem Verhältnis von Destillat/Isopropanol = 1/2,5.

Beispiel 2:

Kontinuierliche Herstellung von Glykolid aus Natriumchloracetat mit katalytischen Mengen Zinkoxid

Natriumchloracetat wird mit 2 Gew.-% Zinkoxid versetzt und in einer geeigneten Mischapparatur wie z.B. einem Rhönradmischer homogenisiert.

| Gehäuse Nr. | 0 | 1 | 2 | 3 | 4 | 5 | 6 | 7 | 8 | 9 |
|---|---|---|---|---|---|---|---|---|---|---|
| Temperatur (°C) | 15 | 130 | 150 | 240 | 280 | 280 | 280 | 280 | 280 | 250 |

Der Extruder wird entsprechend obiger Tabelle (vergl. auch Abb. 1) temperiert, die Brüdenableitung wird auf 180°C, die Vakuumvorlagen zur Aufnahme des Destillats auf 80°C aufgeheizt.

Nach Erreichen der solltemperatur wird der Extruder mit einer Drehzahl von 1,33/sec. und einem Feed von 3,3 kg/Stunde Natriumchloracetat bei einem Druck von 2-5 mbar betrieben.

Die Vakuumvorlagen werden nach jeweils 60 Minuten entleert, das Destillat wird wie in Beispiel 1 beschrieben, aufgearbeitet.

Beispiel 3

Kontinuierliche Herstellung von [S,S]-Lactid aus [S]-Polymilchsäure mit katalytischen Mengen Zinkoxid

[S]-Polymilchsäure wird mit 2 Gew.-% Zinkoxid versetzt und in einer geeigneten Mischapparatur wie z.B. einem Rhönradmischer homogenisiert.

Der Extruder wird segmentweise von 15 bis 285°C temperiert. Die Brüdenableitung wird auf 170°C aufgeheizt, die Temperatur der Vakuumvorlagen zur Aufnahme des Destillats beträgt 90°C. Nach Erreichen der Solltemperatur wird der Extruder mit einer Drehzahl von 2,5/sec und einem Feed von 2,5 kg/Stunde [S]-Polymilchsäure bei einem Druck von 2 - 5 mbar betrieben. Die Vakuumvorlagen werden nach jeweils 60 Minuten entleert, das Destillat wird aufgearbeitet.

Die Ausbeute an Destillat beträgt 95 % d.Th. Die Aufarbeitung des Destillats erfolgt durch satzweises Umfällen aus Isopropanol in einem Verhältnis von 1/1,5 - Destillat / Isopropanol.

Beispiel 4

Kontinuierliche Herstellung von [R,R]-Lactid aus [R]-Polymilchsäure mit katalytischen Mengen Zinkoxid

Die Herstellung des enantiomeren Lactids folgt der gleichen Verfahrenweise wie beim optischen Antipoden -[S,S]-Lactid beschrieben.

Beispiel 5

Kontinuierliche Herstellung von [R,R],[S,S]-Lactid (racemisches Lactid und [R,S]-Lactid (Meso-Lactid aus [R], [S]-Polymilchsäure mit katalytischen Mengen Zinkoxid

Die Herstellung des Gemisches von [R,R],[S,S]-Lactid (racemisches Lactid und [R,S]-Lactid (Meso-Lactid) folgt der gleichen Verfahrensweise wie beim [S,S]-Lactid beschrieben. Die Trennung von racemischem und meso-Lactid erfolgt in einem separaten Aufarbeitungsschritt.

Beispiel 6

Kontinuierliche Destillation von [S,S]-3,6-Dimethyl-1,4-dioxan-2,5-dion

| Gehäuse-Nr. | 0 | 1 | 2 | 3 | 4 | 5 | 6 | 7 | 8 | 9 |
|---|---|---|---|---|---|---|---|---|---|---|
| Temperatur [ ° C] | 15 | 50 | 70 | 140 | 190 | 200 | 200 | 200 | 190 | 190 |

Die Brüdenableitung wird auf 140 ° C temperiert, die Doppelmantelglasvorlagen werden mit heißem Wasser auf 95 ° C beheizt.

Nach Erreichen der Solltemperatur wird der Extruder mit einer Einspeisung von 5 kg pro Stunde [S,S]-3,6-Dimethyl-1,4-dioxan-2,5-dion und einer Schneckendrehzahl von 1,67 1/sec betrieben. Während der Destillation beträgt der Druck 2 - 4 mbar. Das Destillat wird nach Belüftung mit Stickstoff in ein Thermogefäß abgelassen und in einer Universalapparatur im Verhältnis von 1/4 in Petrolether 60/90 gefällt. Der Feststoff wird über eine Zentrifuge abgetrennt und in einem Vakuumtrockenschrank getrocknet.

Die Ausbeute beträgt 95 - 98 % des Einsatzes.

Beispiel 7

Kontinuierliche Destillation von [R,R]-3,6-Dimethyl-1,4-dioxan-2,5-dion

Die Destillation des [R,R] Enantiomeren des 3,6-Dimethyl-1,4-dioxan-2,5-dions folgt der in Beispiel 3 angegebenen Vorschrift für das [S,S]-Enantiomere des Lactids.

Beispiel 8

Kontinuierliche Destillation von [R,R],[S,S]-3,6-Dimethyl-1,4-dioxan-2,5-dion

Die Destillation des Racemats des 3,6-Dimethyl-1,4-dioxan-2,5-dions folgt der in Beispiel 3 angegebenen Vorschrift für das [S,S]-Enantiomere.

Beispiel 9

Kontinuierliche Destillation von [R,S]-3,6-Dimethyl-1,4-dioxan-2,5-dion

Die Destillation des meso 3,6-Dimethyl-1,4-dioxan-2,5-dions folgt der in Beispiel 3 angegebenen Vorschrift für das [S,S]-Enantiomere des Lactids.

Beispiel 10

Kontinuierliche Destillation von 3-Hydroxyacetophenon

| Gehäuse-Nr. | 0 | 1 | 2 | 3 | 4 | 4 | 6 | 7 | 8 | 9 |
|---|---|---|---|---|---|---|---|---|---|---|
| Temperatur [ ° C] | 15 | 25 | 55 | 105 | 180 | 180 | 180 | 180 | 180 | 250 |

Die Brüdenableitung wird auf 170 ° C temperiert, die Doppelmantelglasvorlagen werden mit heißem Wasser auf 90 ° C beheizt.

Nach Erreichen der Solltemperatur wird der Extruder mit einer Einspeisung von 4,3 kg pro Stunde 3-Hydroxyacetophenon und einer Schneckendrehzahl von 2,5 1/sec betrieben. Während dei Destillation beträgt der Druck 2 - 4 mbar. Das Destillat wird nach Belüftung mit Stickstoff in ein Thermogefäß abgelassen und in einer Universalapparatur im Verhältnis von 1/2 in Wasser gefällt. Der Feststoff wird über eine Zentrifuge abgetrennt und in einem Umlufttrockenschrank getrocknet.

Die Ausbeute beträgt 91 % des Einsatzes.

Beispiel 11

Kontinuierliche Destillation von 3-Amino-2-chlorpyridin

| Gehäuse-Nr. | 0 | 1 | 2 | 3 | 4 | 5 | 6 | 7 | 8 | 9 |
|---|---|---|---|---|---|---|---|---|---|---|
| Temperatur [°C] | 15 | 50 | 65 | 70 | 80 | 130 | 130 | 130 | 130 | 120 |

Die Brüdenableitung wird auf 130°C temperiert, die Doppelmantelglasvorlagen werden mit heißem Wasser auf 80°C beheizt.

Nach Erreichen der Solltemperatur wird der Extruder mit einer Einspeisung von 3,2 kg pro Stunde 3-Amino-2-chlorpyridin und einer Schneckendrehzahl von 0,83 l/sec betrieben. Während der Destillation beträgt der Druck 2 - 4 mbar. Das Destillat wird nach Belüftung mit Stickstoff in ein Thermogefäß abgelassen und in einer Universalapparatur im Verhältnis von 1/1,4 l Wasser gefällt. Der Feststoff wird über eine Zentrifuge abgetrennt und in einem Umluftrockenschrank getrocknet. Die Ausbeute beträgt 85 % d.E.

Beispiel 12

Kontinuierliche Destillation von Glycolid (1,4-dioxan-2,5-dion

| Gehäuse-Nr. | 0 | 1 | 2 | 3 | 4 | 5 | 6 | 7 | 8 | 9 |
|---|---|---|---|---|---|---|---|---|---|---|
| Temperatur [°C] | 15 | 50 | 70 | 140 | 190 | 200 | 200 | 200 | 190 | 190 |

Die-Brüdenableitung wird auf 140°C temperiert, die Doppelmantelglasvorlagen werden mit heißem Wasser auf 85°C beheizt. Nach Erreichen der Solltemperatur wird der Extruder mit einer Einspeisung von 5 kg Glykolid pro Stunde und einer Schneckendrehzahl von 1,67 (1/sec) betrieben. Während der Destillation beträgt der Druck 2-4 mbar. Das Destillat wird nach Belüftung mit Stickstoff in ein Thermogefäß abgelassen und in einer 50 l Apparatur im Verhältnis 1 : 4 in Petrolether 60/90 gefällt. Die so erhaltene Kristallsuspension wird über eine Zentrifuge abgetrennt und in einem Vakuumtrockenschrank getrocknet. Die Ausbeute beträgt 95-98 % d. E. Das Material ist z.B. zur Herstellung von Nahtmaterialien geeignet.

Produktbescheibung:

Aussehen: weiße Kristalle
Geruch: fast geruchlos
Identität: IR: α-Form
Löslichkeit: leicht löslich in Aceton, schwer löslich in Toluol
$H_2O$ 0,01 %
(nach Karl Fischer)
Schwermetalle: < 10 ppm
Freie Säure: 0,02 %
Gehalt: 99,8 % - 100,0 %
Schmelzpunkt: 84,1°C
(Differentialthermoanalyse)

**Patentansprüche**

**1.** Verfahren zur Destillation thermolabiler Verbindungen, dadurch gekennzeichnet, daß man

(a) die zu destillierende Verbindung aus einem Vorratsbehälter in das Strömungsrohr eines evakuierbaren selbstreinigenden Doppelschneckenextruders versehen mit getrennt regelbaren Heizsegmenten, mindestens einer Brüdenableitung und einem davon getrennten Austrag, einspeist und mittels Zwangsförderung durch Heizsegmente mit ansteigender Temperatur auf Siedetemperatur erhitzt,

(b) im Bereich der Siedetemperatur das Destillat über die Brüdenableitung(en) abzieht, anschließend kondensiert und

(c) den höhersiedenden Rückstand durch Zwangsförderung aus dem Destillationsbereich in einen

Rückstandsbehälter austrägt.

2. Verfahren nach Anspruch 1, dadurch gekennzeichnet, daß die Heizsegmente zwischen 15 und 400 ° C temperiert sind.

3. Verfahren nach einem der Ansprüche 1 bis 2, dadurch gekennzeichnet, daß die Temperatur der Heizsegmente nach den Brüdenableitungen niedriger ist als unterhalb der Brüdenableitungen.

4. Verwendung eines evakuierbaren Doppelschneckenextruders, versehen mit getrennt regelbaren Heizsegmenten, mindestens einer Brüdenableitung für das Destillat und davon getrenntem Austrag für den Destillationsrückstand zur Destillation bei der Herstellung oder Reinigung thermolabiler Verbindungen.

5. Verfahren zur Herstellung von einem Glycolid der allgemeinen Formel

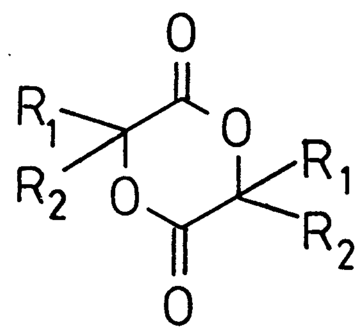

worin $R_1$ und $R_2$ unabhängig voneinander Wasserstoff oder einen verzweigten oder unverzweigten Alkylrest mit bis zu 16 Kohlenstoffatomen bedeuten können, durch Thermolyse der entsprechenden niedermolekularen Poly-$\alpha$-hydroxycarbonsäure oder des aus einem $\alpha$-Halogencarbonsäuresalz in situ gebildeten Polyesters in Gegenwart eines Metalls, Metalloxids oder Salzes als Katalysator, dadurch gekennzeichnet, daß man

(a) die zu thermolysierende Verbindung aus einem Vorratsbehälter in das Strömungsrohr eines evakuierbaren, selbstreinigenden Doppelschneckenextruders, versehen mit getrennt regelbaren Heizsegmenten, mindestens einer Brüdenableitung und einem davon getrennten Austrag, einspeist und mittels Zwangsförderung durch Heizsegmente mit ansteigender Temperatur auf Siedetemperatur, gegebenenfalls unter Schutzgas und/oder vermindertem Druck erhitzt,

(b) im Bereich der Siedetemperatur das Destillat über die Brüdenableitung(en) abzieht, anschließend kondensiert und

(c) den höhersiedenden Rückstand durch Zwangsförderung aus dem Destillationsbereich in einen Rückstandsbehälter austrägt.

6. Verfahren nach Anspruch 5, dadurch gekennzeichnet, daß der Temperaturgradient von 15 bis 280 ° C ansteigt und der Druck 0.1 bis 5 mbar beträgt.

7. Verfahren zur Herstellung von 1,4-Dioxan-2,5-dion nach Anspruch 5, dadurch gekennzeichnet, daß als Ausgangsverbindung niedermolekulare Polyglycolide oder Halogenessigsäuresalze in Gegenwart eines Katalysators eingesetzt werden.

8. Poly-(1,4-dioxan-2,5-dion), dadurch gekennzeichnet, daß es eine inhärente Viskosität von > 1,4 [dl/g], gemessen in Hexafluorisopropanol bei 30 ° C, aufweist.

**Claims**

1. A process for distilling thermolabile compounds, characterised in that

(a) the compound to be distilled is taken from a storage container and fed into the flow tube of an evacuable self-cleaning double screw extruder equipped with heating segments able to be separately regulated, at least one vapour discharge pipe and an outlet which is separate from it, and is

heated to boiling point by being forced to flow through heating segments at increasing temperatures,
(b) in the region of the boiling temperature the distillate is drawn off through the vapour discharge pipe(s), then condensed and
(c) the higher-boiling residue is removed by forced flow from the distillation area into a residue container.

2. A process according to claim 1, characterised in that the heating segments are adjusted to temperatures between 15 and 400° C.

3. A process according to one of claims 1 to 2, characterised in that the temperature of the heating segments after the vapour discharge pipes is lower than below the vapour discharge pipes.

4. Use of an evacuable double screw extruder, equipped with heating segments able to be separately regulated, at least one vapour discharge pipe for the distillate and a separate outlet for the distillation residue for distillation in the production or purification of thermolabile compounds.

5. A process for preparing a glycolide of general formula

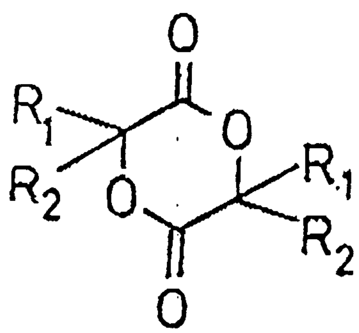

wherein $R_1$ and $R_2$ independently of each other may represent hydrogen or a branched or unbranched alkyl group having up to 16 carbon atoms, by thermolysis of the corresponding lower-molecular weight poly-α-hydroxycarboxylic acid or the polyester formed in situ from an α-halocarboxylic acid salt, in the presence of a metal, metal oxide or salt as catalyst, characterised in that
(a) the compound to be thermolysed is taken from a storage container and fed into the flow tube of an evacuable, self-cleaning double-screw extruder, equipped with heating segments able to be separately regulated, at least one vapour discharge pipe and a separate outlet, and is heated to boiling temperature by being forced to flow through heating segments at increasing temperatures, optionally under protective gas and/or reduced pressure,
(b) in the region or the boiling temperature the distillate is drawn off through the vapour discharge pipe(s), then condensed and
(c) the higher-boiling residue is removed by forced flow from the distillation area into a residue container.

6. A process according to claim 5, characterised in that the temperature gradient rises from 15 to 280° C and the pressure is from 0.1 to 5 mbar.

7. A process for preparing 1,4-dioxane-2,5-dione according to claim 5, characterised in that lower-molecular weight polyglycolides or haloacetic acid salts in the presence of a catalyst are used as the starting compound.

8. Poly-(1,4-dioxane-2,5-dione), characterised in that it has been an inherent viscosity of >1.4 [dl/g], measured in hexafluoroisopropanol at 30° C.

## Revendications

1. Procédé de distillation de composés thermolabiles, caractérisé en ce que
(a) on fait passer le composé à distiller d'un réservoir de stockage dans le tube d'écoulement d'une

boudineuse à deux vis autonettoyante susceptible d'être mise sous vide munie de segments chauffants réglables séparément, d'au moins une conduite d'évacuation des vapeurs et d'une sortie séparée de celle-ci, et on chauffe à la température d'ébullition par transport forcé à travers les segments chauffants de température croissante,

(b) on soutire le distillat par la ou les conduites d'évacuation des vapeurs dans le domaine de la température d'ébullition, puis on le condense et

(c) on extrait du domaine de distillation le résidu à plus haut point d'ébullition par transport forcé pour l'amener dans un réservoir de résidu.

**2.** Procédé selon la revendication 1, caractérisé en ce que les segments chauffants sont équilibrés en température entre 15 et 400 ° C.

**3.** Procédé selon l'une des revendications 1 à 2, caractérisé en ce que la température des segments chauffants en aval des conduites d'évacuation des vapeurs est plus basse qu'au dessous des conduites d'évacuation des vapeurs.

**4.** Utilisation d'une boudineuse à deux vis susceptible d'être mise sous vide, munie de segments chauffants réglables séparément, d'au moins une conduite d'évacuation des vapeurs pour le distillat et d'une sortie séparée de celle-ci pour le résidu de distillation pour la distillation lors de la préparation ou de la purification de composés thermolabiles.

**5.** Procédé de préparation d'un glycolide de formule générale

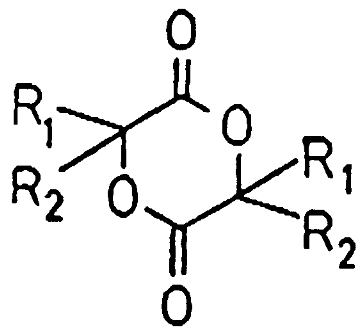

dans laquelle $R_1$ et $R_2$ peuvent représenter indépendamment l'un de l'autre un atome d'hydrogène ou un reste alkyle ramifié ou non ramifié ayant jusqu'à 16 atomes de carbone, par thermolyse du poly-(acide $\alpha$-hydroxycarboxylique) de faible poids moléculaire correspondant ou du polyester formé in situ à partir d'un sel d'acide $\alpha$-halogénocarboxylique en présence d'un métal, oxyde métallique ou sel en tant que catalyseur, caractérisé en ce que

(a) on fait passer le composé à thermolyser d'un réservoir de stockage dans le tube d'écoulement d'une boudineuse à deux vis autonettoyante susceptible d'être mise sous vide, munie de segments chauffants réglables séparément, d'au moins une conduite d'évacuation des vapeurs et d'une sortie séparée de celle-ci, et on chauffe à la température d'ébullition par transport forcé à travers les segments chauffants de température croissante, éventuellement sous gaz protecteur et/ou sous pression réduite,

(b) on soutire le distillat par la ou les conduites d'évacuation des vapeurs dans le domaine de la température d'ébullition, puis on le condense et

(c) on extrait du domaine de distillation le résidu à plus haut point d'ébullition par transport forcé pour l'amener dans un réservoir de résidu.

**6.** Procédé selon la revendication 5, caractérisé en ce que le gradient de température monte de 15 à 580 ° C et la pression est de 0,1 à 5 mbar.

**7.** Procédé de préparation de la 1,4-dioxanne-2,5-dione selon la revendication 5, caractérisé en ce que l'on utilise comme composé de départ des polyglycolides de faible poids moléculaire ou des sels d'acide halogénoacétique en présence d'un catalyseur.

**8.** Poly(1,4-dioxanne-2,5-dione), caractérisée en ce qu'elle a une viscosité intrinsèque, mesurée dans l'hexafluoroisopropanol à 30 ° C, > 1,4 [dl/g].

Abb. 1.

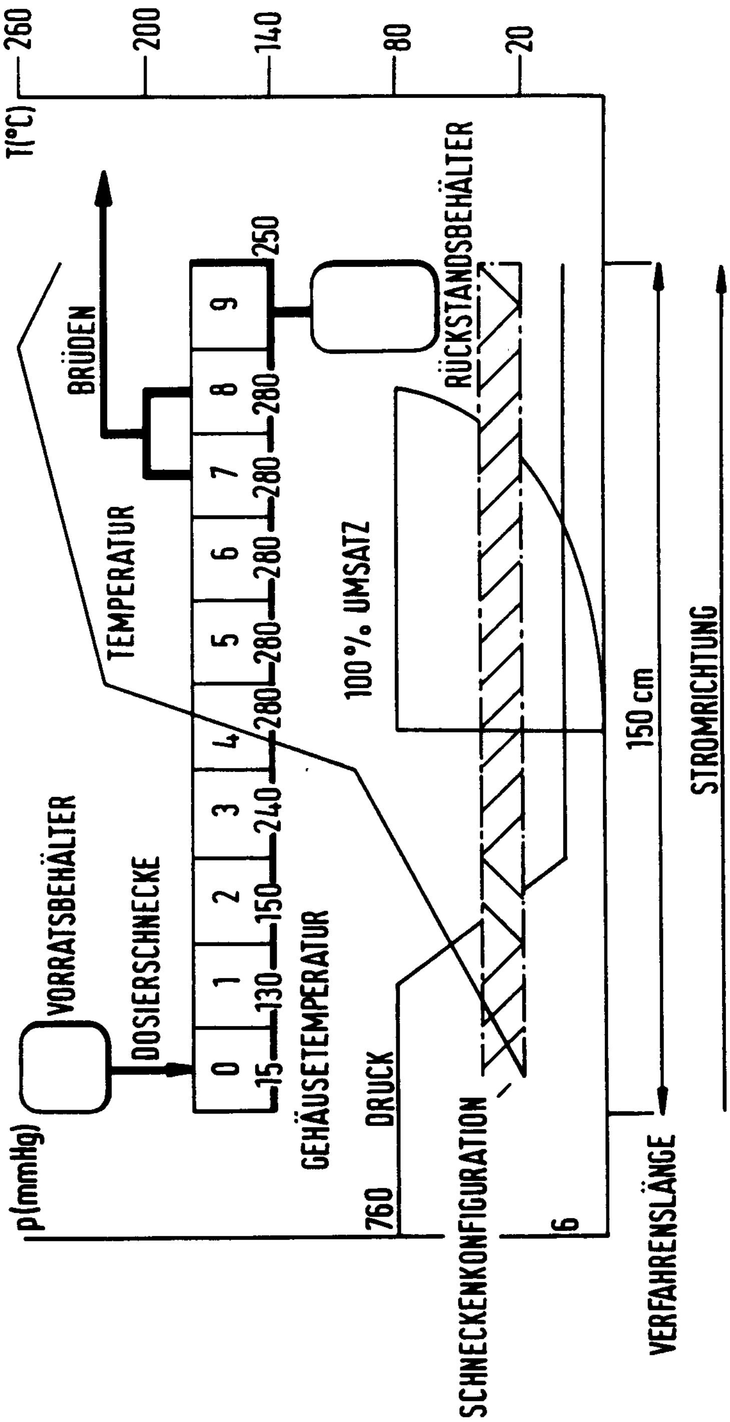
p(mmHg)
760
6
VORRATSBEHÄLTER
DOSIERSCHNECKE
0
1
2
3
4
5
6
7
8
9
15
130
150
240
280
280
280
280
280
250
GEHÄUSETEMPERATUR
DRUCK
TEMPERATUR
BRÜDEN
100 % UMSATZ
RÜCKSTANDSBEHÄLTER
SCHNECKENKONFIGURATION
VERFAHRENSLÄNGE
150 cm
STROMRICHTUNG
T(°C)
260
200
140
80
20

Abb. 2.

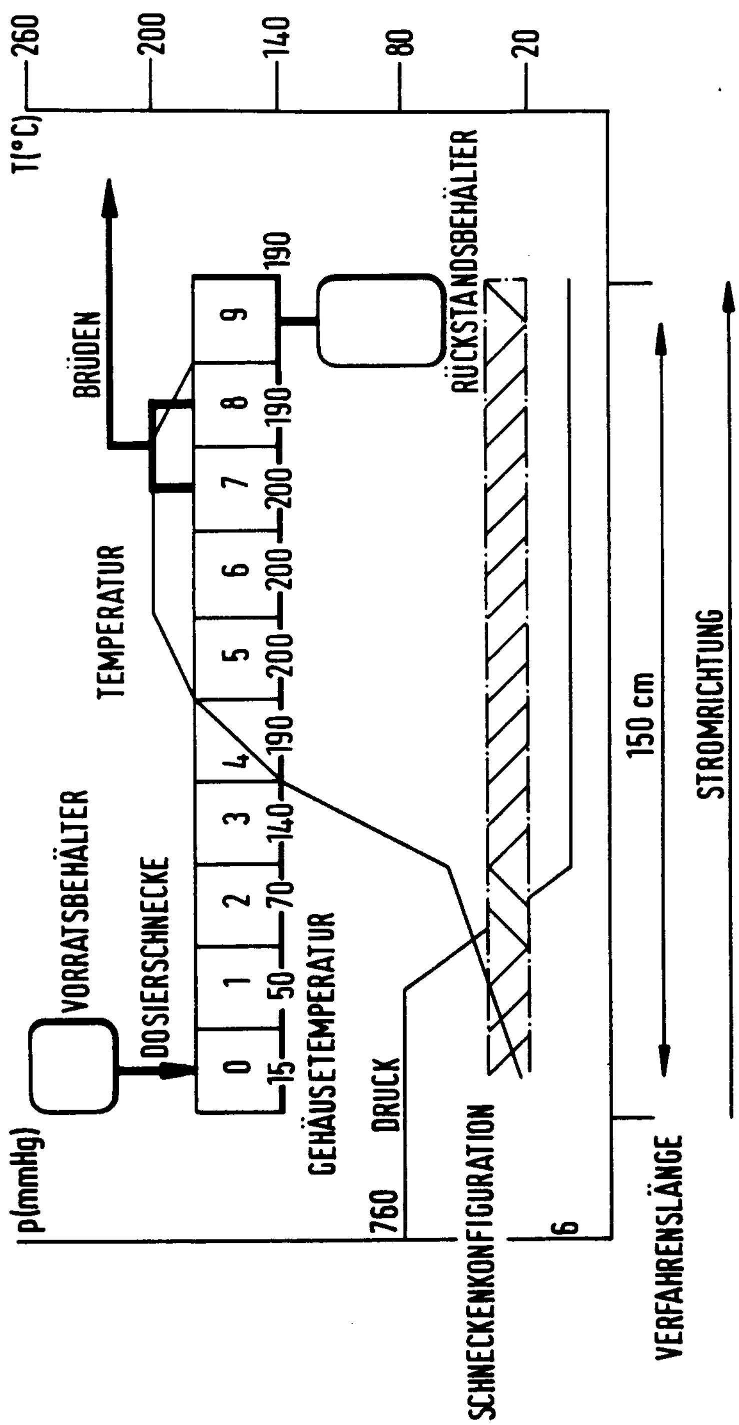
T(°C)
260
200
140
80
20
p(mmHg)
760
6
VORRATSBEHÄLTER
DOSIERSCHNECKE
0
1
2
3
4
5
6
7
8
9
15
50
70
140
190
200
200
200
190
190
GEHÄUSETEMPERATUR
TEMPERATUR
DRUCK
BRÜDEN
RÜCKSTANDSBEHÄLTER
SCHNECKENKONFIGURATION
150 cm
STROMRICHTUNG
VERFAHRENSLÄNGE